(19) Europäisches Patentamt
European Patent Office
Office européen des brevets

(11) Publication number: **0 202 823**
**A2**

# (12) EUROPEAN PATENT APPLICATION

(21) Application number: 86303501.0

(51) Int. Cl.[4]: C 07 D 239/66

(22) Date of filing: 08.05.86

(30) Priority: 17.05.85 US 735895

(43) Date of publication of application: 26.11.86 Bulletin 86/48

(84) Designated Contracting States: AT BE CH DE FR GB IT LI LU NL SE

(71) Applicant: UNIROYAL LIMITED
1500 Don Mills Road
Don Mills Ontario M3B 3L4(CA)

(72) Inventor: Brewer, Arthur D.
RR1 Puslinch
Wellington Ontario N0B 2J7(CA)

(74) Representative: Harrison, Michael Robert et al,
Urquhart-Dykes & Lord 5th Floor Tower House Merrion Way
Leeds, LS2 8PA(GB)

## (54) Purification of 5-pyrimidinecarboxamides.

(57) A process for the purification of 5-pyrimidinecarboxamides to highly purified states which are essentially insoluble in both organic and inorganic solvents comprises contact with an organic base to form an adduct. The adduct is purified through recrystallization and the desired 5-pyrimidinecarboxamide is regenerated from the adduct in highly purified form with acid.

Additionally, an adduct resulting from the combination of a 5-pyrimidinecarboxamide with an organic base is provided, which adduct is capable of being purified using recrystallization techniques, whereas the 5-pyrimidinecarboxamide is not itself capable of being so purified due to its relative insolubility in organic and inorganic solvents.



Croydon Printing Company Ltd.

0202823

# PURIFICATION OF 5-PYRIMIDINECARBOXAMIDES

## Background of the Invention

This invention relates to the purification of 5-Pyrimidinecarboxamides such as the 5-carboxamido or 5-thiocarboxamido derivatives of 2-thio- or 2-selenobarbituric acid disclosed in copending U.S. patent application Serial No. 699,720 filed on February 8, 1985 [593 KON-IIA], or the N-phenyl-5-carboxamido-2 thiobarbituric acid derivatives disclosed in copending U.S. patent application Serial No. 699,776 filed on February 8, 1985 [599 KON-IA].

5-Pyrimidinecarboxamides and related compounds have been purified using conventional techniques such as column chromatography or recrystallization. For example, U.S. Patent No. 3,784,547 (Samour et al), U.S. Patent No. 3,999,974 (Hirono et al), U.S. Patent No. 4,460,588 (Serban et al) and Japanese Patent Publication No. 1445/64 [39(1964)-1445] disclose such techniques for purifying various 5-substituted barbituric acids and other pyrimidine derivatives.

The 5-Pyrimidinecarboxamides described in the aforesaid copending applications exhibit anti-leukemia and anti-tumor activity, and thus have potential pharmaceutical application. It has not, however, previously been possible to obtain such materials in chemically pure states. To the contrary, these compounds are difficult to handle and process, due to their extreme insolubility in organic or inorganic solvents. That insolubility precludes purification by recrystallization techniques such as described in the above patents or otherwise commonly utilized in preparative organic chemistry. Furthermore, these compounds are not easily purified by chromatographic techniques, which are in any case only applicable on a small scale, require expensive apparatus and highly trained personnel, and are of marginal efficacy and poor productivity. Since an exceptional degree of purity is mandated for an agent which is used therapeutically on human patients, the difficulty of purification of the

5-Pyrimidinecarboxamides represents a serious problem in their development as anti-cancer drugs.

It is accordingly among the objects of the present invention to provide an improved process for the purification of 5-Pyrimidinecarboxamides, particularly those of the aforesaid copending applications, by which the desired compounds may be obtained in very high, pharmaceutical-grade purity on a bulk scale. Other objects and advantages of the invention will be apparent from the following detailed description of preferred forms of the invention.

## Summary of the Invention

The purification process of the present invention is applicable to purification of the 5-Pyrimidinecarboxamides disclosed in the aforesaid copending applications, particularly 5-carboxamido-2-thiobarbituric acid derivatives described in the aforesaid application Serial No. 699,776. These compounds have the formula:

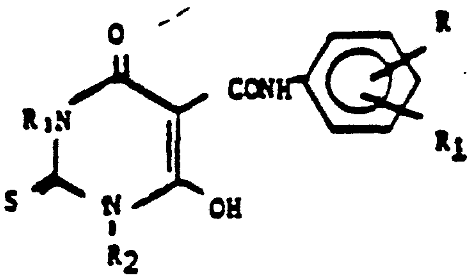

wherein

R is hydrogen, 2 or 3-halo, 2-methyl, 4-fluoro, 4-($C_1$-$C_6$ alkoxyl), 2 or 4-trifluoromethyl, or hydroxyl, and $R_1$ is hydrogen; or

R is 2-fluoro and $R_1$ is 4-fluoro; or

R is 2-methoxy and $R_1$ is 5-methyl; and

$R_2$ and $R_3$ are hydrogen atoms or carbohydrate residues.

The carbohydrate residues may be furanosyl (e.g., ribofuranosyl), pyranosyl (e.g., arabinopyranosyl, glucopyranosyl, or galactopyranosyl), their deoxy derivatives, or their aliphatic analogs (e.g., hydroxyalkoxyalkyl or polyhydroxyalkyl groups having from 2 to 12 carbon atoms in each of the alkoxy and alkyl moieties thereof, such as 2-hydroxyethoxymethyl or 2,3-dihydroxypropyl. As used herein, the term "carbohydrate residue" is intended to refer to those cyclic and acyclic groups which form pyrimidine nucleosides or the psuedo nucleosides, e.g., materials including both the cyclic and acyclic groups specified hereinabove.

The 5-carboxamide-2-thiobarbituric acid derivatives purified in accordance with the invention can exist in the form illustrated in the above formula or in any of its tautomeric forms.

In accordance with the invention, these 5-Pyrimidinecarboxamides are purified by reacting them with a suitable organic base to form a corresponding adduct, purifying the adduct, and regenerating the 5-Pyrimidinecarboxamide from the purified adduct with acid. Surprisingly, adducts thus formed are readily purified, e.g., by recrystallization one or more times from a solvent in which there is substantial differential solubility with temperature, and the original carboxamides regenerated, in substantially chemically pure state. Moreover, the purification may be effected on a large scale without the necessity of high capital investment or processing costs.

Description of the Preferred Embodiments

In accordance with a preferred embodiment of the invention, a 5-Pyrimidinecarboxamide is initially reacted with a suitable organic base to form an adduct. The organic base is an alkylamine or alkanolamine having the formula R'R"R'''N, wherein at least one of R', R" and R''' is an alkyl or hydroxyalkyl group having from 1 to 24 carbon atoms (preferably $C_{1-12}$), or two or three of the R', R" and R''' groups form a basic nitrogen-containing heterocyclic moiety, and the remaining substituents are hydrogen. Examples of organic amines which may be so utilized include monoalkylamines such as dodecylamine; trialkylamines such as triethylamine; trialkanolamines such as triethanolamine and basic nitrogen-containing heretocyclic amines such as 2,6-dimethylmorpholine or piperidine.

The 5-Pyrimidinecarboxamide and the base are suitably reacted in molar proportions varying from about 1:1 to 1:5, preferably from about 1:2 to 1:3, carboxamide to base. One-to-one adducts of the carboxamide are thereby formed.

The reaction is carried out by initially suspending the 5-Pyrimidinecarboxamide in a relatively polar organic solvent. Solvents such as the lower ($C_1$-$C_5$) alcohols (e.g., ethanol, lower aliphatic ketones (e.g., methyl isobutyl ketone), heterocyclic ethers (e.g., 1,4-dioxane), heterocyclic solvents (e.g., pyridine or sulfolane), or mixtures thereof (e.g., ethanol and pyridine) have been found so useful.

The suspension is thereafter warmed and the organic base is added, forming the desired adduct which is partially soluble in the suspension. The suspension is then brought to boiling, desirably under gentle reflux, and additional solvent is added until all solids go into solution.

The adduct is thereafter purified by recrystallization. This is accomplished by cooling the adduct/solvent mixture until the purified adduct

crystallizes, generally at a temperature of from about +20°C to -20°C.

Finally, the purified adduct is treated with acid to regenerate the purified 5-Pyrimidinecarboxamide therefrom. Preferably, the regeneration is accomplished by triturating the purified adduct crystals with acid (e.g., hydrochloric, sulfuric, nitric, acetic, or other mineral acid), and then washing the regenerated crystals with water. The purity of the regenerated 5-Pyrimidinecarboxamide may then be determined, and the process repeated if a higher purity product is required.

Depending on the 5-Pyrimidinecarboxamide treated improved purities may be obtained by alternating the organic base and/or the solvent used during successive repetitions of the purification operations.

It is particularly preferred to utilize the process of the invention for the purification of N-substituted phenyl-5-carboxamido-2-thiobarbituric acid, viz., 1,2,3,4-tetrahydro-6-hydroxy-4-oxo-N-phenyl-2-thioxo-5-pyrimidinecarboxamide. Employing the present technique, that compound can be refined to purities approaching 100%. The following examples illustrate, but do not restrict, the purification of this and similar 5-Pyrimidinecarboxamides, employing this process. Unless otherwise indicated, all parts and percentages specified in the examples are by weight, and all temperatures in degrees Celsius.

Example 1

A sample of 1,2,3,4-tetrahydro-6-hydroxy-4-oxo-N-phenyl-2-thioxo-5-pyrimidinecarboxamide was prepared as described in Example 1A of the aforesaid copending application Serial No. 699,766. By high pressure liquid chromatography (HPLC) this sample was found to contain 96.1% of the carboxamide, i.e., it had a purity of 96.1%; three other unidentified components (A,B and C) were present as impurities in amounts of 0.8, 2.7 and 0.4% by weight, respectively.

A sample of the semi-pure carboxamide was

suspended in absolute ethanol, two equivalents of triethylamine were added, and the suspension was boiled, with the addition of ethanol, until all solids went into solution. On cooling to a temperature of 0°C, a crystalline solid appeared which was collected, washed with a little ethanol and triturated with dilute hydrochloric acid. The purified product was washed with water and dried; the desired carboxamide was obtained in 98.8% purity. The process was repeated twice, producing an intermediate product having a 99.2% purity and a final product having 99.7% purity.

Example 2

Using the same semi-pure specimen as in Example 1, the procedure described in Example 1 was repeated, using as the recrystallizing solvent a 50:50 mixture (by weight) of ethanol and pyridine. Two successive products having purities of 99.7% and 99.8% of the desired carboxamide were thus formed.

Example 3

The procedure of Example 1 was followed with the same initial specimen, using two equivalents of triethanolamine in place of triethylamine. Successive products having 99.99% and 100% purities were thus obtained.

Example 4

The procedure of Example 1 was followed with the same specimen, using two equivalents of n-dodecylamine in place of triethylamine. After a single purification, a product containing 99.96% of the carboxamide was obtained.

Example 5

A further sample of 1,2,3,4-tetrahydro-6-hydroxy-4-oxo-N-phenyl-2-thioxo-5-pyrimidinecarboxamide was prepared as described in Example 1, but from a different batch; the purity, as assayed by HPLC, was 93.6%. This specimen was then purified in the manner described in Example 1 above, using triethylamine to form the adduct initially crystallized, and N-dodecylamine to form a second adduct thereafter

crystallized. The product formed by trituration of the first adduct with HCl contained 99.94% of the carboxamide, while the product formed by trituration of the second adduct with HCl had a 99.98% purity.

Example 6

Using a portion of the same specimen treated in Example 5, the purification procedure described therein was repeated, using N-dodecylamine as the base to form the first adduct and triethylamine to form the second adduct. The successive products had purities of 99.90% and 99.95%.

Example 7

A specimen of 1,2,3,4-tetrahydro-6-hydroxy-N-(2-methylphenyl-4-oxo-2-thioxo-5-pyrimidinecarboxamide was prepared in the manner described in Example 3 of copending application Serial No. 699,766. The crude material had a 73.8% purity. It was treated with triethylamine and ethanol as described in Example 1. The purified product was chemically pure (100% purity).

Example 8

A sample of 1,2,3,4-tetrahydro-6-hydroxy-N-(4-methoxyphenyl)-4-oxo-2-thioxo-5-pyrimidinecarboxamide was prepared as described in Example 6 of the aforesaid copending application. The sample was purified as described in Example 1 above except that dilute, 2 N sulfuric acid was subsituted for hydrochloric acid. The product purity, determined by HPLC, was 100%.

Example 9

A further sample of 1,2,3,4-tetrahydro-6-hydroxy-N-phenyl-4-oxo-2-thioxo-5-pyrimidinecarboxamide was prepared, in the same manner as previously described. The specimen treated was 98% pure. The specimen was suspended in ethanol (10 ml per gram), the suspension was warmed to 50°C, and triethylamine was added (1g per gram of the semi-pure specimen). The suspension was gently refluxed with stirring for 2 hours,and then reduced in volume *in vacuo* to a solid. The suspension was then recrystallized from boiling ethanol; the hot solution was filtered and chilled, giving a crop

of greenish needles which were collected and dried. This solid was thoroughly triturated with an excess of dilute (3 N) HCl. The white solids so produced were filtered and washed with water until the washings no longer gave an acid reaction. It was then dried. The resulting product was substantially pure.

The following table summarizes the analyses of the specimens treated and the various intermediate and final purified products formed in the above examples, the various impurities detected (by HPLC) being identified as Impurities A-E, respectively:

## ANALYSES OF PURIFICATION EXAMPLES

| | Carboxamide Content (Wt.%) [Purity] | Impurity Content (Wt. %) A | B | C | D | E |
|---|---|---|---|---|---|---|
| **Example 1** | | | | | | |
| Raw Specimen | 96.1 | 0.8 | 2.7 | 0.4 | - | - |
| First Intermediate Purified Product | 98.8 | 0.6 | 0.3 | 0.3 | - | - |
| Second Intermediate Purified Product | 99.2 | 0.3 | 0.2 | 0.3 | - | - |
| Final Purified Product | 99.7 | - | - | 0.3 | - | - |
| **Example 2** | | | | | | |
| Raw Specimen | 96.1 | 0.8 | 2.7 | 0.4 | - | - |
| Intermediate Purified Product | 99.7 | - | - | 0.3 | - | - |
| Final Intermediate Purified Product | 99.8 | - | - | 0.2 | - | - |
| **Example 3** | | | | | | |
| Raw Specimen | 96.1 | 0.8 | 2.7 | 0.4 | - | - |
| Intermediate Purified Product | 99.9 | - | 0.01 | - | - | - |
| Final Intermediate Purified Product | 100 | - | - | - | - | - |
| **Example 4** | | | | | | |
| Raw Specimen | 96.1 | 0.8 | 2.7 | 0.4 | - | - |
| Final Purified Product | 99.96 | - | - | 0.04 | - | - |
| **Example 5** | | | | | | |
| Raw Specimen | 93.6 | - | 6.4 | - | - | - |
| Intermediate Purified Product | 99.94 | - | 0.06 | - | - | - |
| Final Intermediate Purified Product | 99.98 | - | 0.02 | - | - | - |
| **Example 6** | | | | | | |
| Raw Specimen | 93.6 | - | 6.4 | | | |
| Intermediate Purified Product | 99.90 | - | 0.10 | - | - | - |
| Final Intermediate Purified Product | 99.95 | - | 0.05 | - | - | - |
| **Example 7** | | | | | | |
| Raw Specimen | 73.8 | 1.6 | 24.4 | - | 0.2 | - |
| Intermediate Purified Product | 100 | - | - | - | - | - |

## ANALYSES OF PURIFICATION EXAMPLES

| | Carboxamide Content (Wt.%) [Purity] | Impurity Content (Wt. %) | | | | |
|---|---|---|---|---|---|---|
| | | A | B | C | D | E |
| **Example 8** | | | | | | |
| Raw Specimen | 99.5 | - | - | - | - | ( |
| Purified Product | 100 | - | - | - | - | |

CLAIMS:

1. A process for the purification of a 5-pyrimidinecarboxamide, characterised in that it comprises the steps of

(a) reacting the 5-pyrimidinecarboxamide with an organic base to form an adduct,

(b) purifying the adduct, and

(c) regenerating the 5-pyrimidinecarboxamide from the purified adduct to obtain the purified 5-pyrimidinecarboxamide.

2. A process according to claim 1, characterised in that the adduct is purified by recrystallization from a solvent.

3. A process according to claim 2, characterised in that the 5-pyrimidinecarboxamide is a 5-carboxamido-2-thiobarbituric acid derivative of the formula:

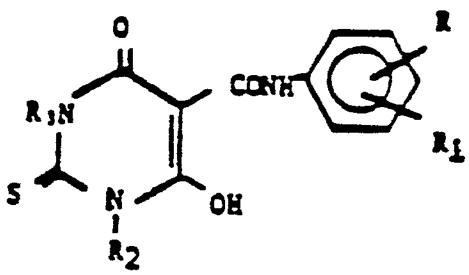

wherein

R is hydrogen, 2 or 3-halo, 2-methyl, 4-fluoro, 4-($C_1$-$C_6$ alkoxyl), 2 or 4-trifluoromethyl, or hydroxyl, and Rl is hydrogen; or

R is 2-fluoro and $R_1$ is 4-fluoro; or

R is 2-methoxy and $R_1$ is 5-methyl; and

$R_2$ and $R_3$ are hydrogen atoms or carbohydrate residues.

4. A process according to any of the preceding claims, characterised in that the 5-pyrimidinecarboxamide

is 1,2,3,4-tetrahydro-6-hydroxy-4-oxo-N-phenyl-2-thioxo-5-pyrimidinecarboxamide, 1,2,3,4-tetrahydro-6-hydroxy-N-(2-methylphenyl)-4-oxo-2-thioxo-5-pyrimidinecarboxamide, 1,2,3,4-tetrahydro-6-hydroxy-N-(4-methoxyphenyl)-4-oxo-2-thioxo-5-pyrimidinecarboxamide.

5. A process according to any of the preceding claims, characterised in that:

step (A) comprises suspending the 5-pyrimidinecarboxamide in a solvent, adding an organic base of the formula R'R"R'''N, wherein at least one of R', R" and R''' is an alkyl or hydroxyalkyl group having from 1 to 24 carbon atoms, or two or three of the R', R" and R''' groups form a basic nitrogen-containing heterocyclic moiety, and the remaining substituents are hydrogen, to the resulting suspension while stirring, and warming the suspension to form a 1:1 adduct with the 5-pyrimidinecarboxamide, heating the suspension, and dissolving the adduct in a solvent,

step (b) comprises cooling the solvent/adduct mixture to crystallize the adduct, and isolating the adduct crystals, and

step (c) comprises triturating the adduct crystals with an acid, and recovering the 5-pyrimidinecarboxamide crystals.

6. A process according to claim 5, characterised in that the solvent is a material in which the adduct is substantially more soluble when the solvent is heated than under ambient conditions.

7. An adduct of a 5-pyrimidinecarboxamide and an organic base characterised in that the 5-pyrimidinecarboxamide is a 5-carboxamide-2-thiobarbituric acid derivative of the formula:

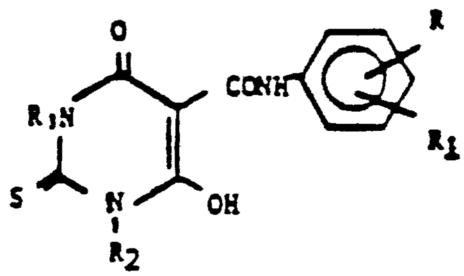

wherein

R is hydrogen, 2 or 3-halo, 2-methyl, 4-fluoro, 4-($C_1$-$C_6$ alkoxyl), 2 or 4-trifluoro-methyl, or hydroxyl, and $R_1$ is hydrogen; or

R is 2-fluoro and $R_1$ is 4-fluoro; and

R is 2-methoxy and $R_1$ is 5-methyl; and

$R_2$ and $R_3$ are hydrogen atoms or carbohydrate residues.

8. An adduct according to claim 7, characterised in that the organic base is an alkylamine or alkanolamine having the formula R'R"R'''N, wherein at least one of R', R" and R''' is an alkyl or hydroxyakyl group having from 1 to 12 carbon atoms, or two or three of the R', R" and R''' groups form a basic nitrogen-containing heterocyclic moiety, and the remaining substituents are hydrogen.

9. An adduct according to claim 8, characterised in that the organic base is triethylamine, triethanolamine, dodecylamine, 2,6-dimethylmorpholine or piperidine.